# EUROPEAN PATENT SPECIFICATION

(11) **EP 3 212 260 B1**
(45) Date of publication and mention of the grant of the patent: **12.05.2021**
(21) Application number: 15784489.5
(22) Date of filing: 15.10.2015
(51) Int. Cl.: A61M 16/00

(54) **CONTROLLING INSUFFLATION VOLUME DURING IN-EXSUFFLATION**
STEUERUNG DES INSUFFLATIONSVOLUMENS WÄHREND IN-EXSUFFLATION
RÉGULATION DU VOLUME D'INSUFFLATION PENDANT L'INSUFFLATION-EXSUFFLATION

(30) Priority: 29.10.2014 US 201462072065 P
(43) Date of publication of application: 06.09.2017
(73) Proprietor: Koninklijke Philips N.V., 5656 AG Eindhoven (NL)
(72) Inventor: LEE, Seunghyun, 5656 AE Eindhoven (NL)
(74) Representative: Philips Intellectual Property & Standards
(86) International application number: PCT/IB2015/057907
(87) International publication number: WO 2016/067146

(56) References cited:
- WO-A1-2012/080892
- WO-A1-2013/140333
- WO-A1-2013/182944
- WO-A1-2014/096996
- WO-A2-2010/058308
- WO-A2-2012/085787

## Description

### CROSS-REFERENCE TO RELATED APPLICATIONS

This patent application claims the priority benefit under 35 U.S.C. § 119(e) of U.S. Provisional Application No. 62/072,065, filed on October 29, 2014.

### BACKGROUND OF THE INVENTION

### 1. Field of the Invention

The present disclosure pertains to a system and method to in-exsufflate a subject.

### 2. Description of the Related Art

It is well known to provide mechanical in-exsufflation (M I-E) therapy that is controlled with pressure and time settings. Clinicians are often faced with a dilemma between the effectiveness and the safety of M I-E therapy when determining an optimum pressure setting for their patients. The primary goal of the insufflation phase of the M I-E therapy is to achieve the maximum lung recruitment without over distension of the lung. Clinicians usually rely on their experiences, observation of the patient's chest expansion, and limited patient feedback to determine whether or not the insufflation pressure level and the insufflation time are set appropriately.

The over estimation of the insufflation pressure and time may result in over distension of the lung, causing potential harm to the patient. On the other hand, the under estimation of the insufflation pressure and time may result in poor lung recruitment, which can compromise the effectiveness of the M I-E therapy.

### SUMMARY OF THE INVENTION

The present invention relates to a system configured to inexsufflate a subject according to claim 1. The system comprises means for generating a pressurized flow of breathable gas for delivery to an airway of the subject; means for generating output signals conveying information related to one or more gas parameters of the pressurized flow of breathable gas; means for obtaining a target respiratory volume; means for determining one or more gas parameters of the pressurized flow of breathable gas; means for delivering in-exsufflation therapy during a first respiratory cycle according to an in-exsufflation therapy regime based on the determined gas parameters, the first respiratory cycle including a first insufflation and a first exsufflation; means for determining one or more breathing parameters of the subject based on the output signals during the first respiratory cycle; and means for delivering in-exsufflation therapy during a second respiratory cycle according to an in-exsufflation therapy regime based on the determined breathing parameters of the subject and the target respiratory volume. The means for determining one or more breathing parameters of the subject is further configured to determine one or more of a forced vital capacity, an inspiratory reserve volume, an inspiratory capacity, an expiratory reserve volume, or an upper inflection point.

WO2013/182944 describes a method and system configured to in-exsufflate a subject by controlling the in-exsufflation pressure waveform. The system includes a control module configured to control a pressure generator to modulate the pressure of the pressurized flow of breathable gas within a respiratory phase (inhalation or exhalation) to generate a percussive waveform within the respiratory phase. The pressure generator is controlled based on gas parameters or breathing parameters determined by a parameter module. The control module monitors the responsiveness of the flow rate of breathable gas to applied modulations in pressure.

These and other objects, features, and characteristics of the present disclosure, as well as the methods of operation and functions of the related elements of structure and the combination of parts and economies of manufacture, will become more apparent upon consideration of the following description and the appended claims with reference to the accompanying drawings, all of which form a part of this specification, wherein like reference numerals designate corresponding parts in the various figures. It is to be expressly understood, however, that the drawings are for the purpose of illustration and description only and are not intended as a definition of the limits of the disclosure.

### BRIEF DESCRIPTION OF THE DRAWINGS

FIG. 1 schematically illustrates a system configured to inexsufflate a subject;
FIG. 2 illustrates vital capacity volume versus target volume profile during volume mode in-exsufflation therapy;
FIG. 3 illustrates lung volume compartments during mechanical in-exsufflation therapy;
FIG. 4 illustrates an estimation of the upper inflection point using mechanical in-exsufflation therapy; and
FIG. 5 illustrates a method for inexsufflating a subject.

### DETAILED DESCRIPTION OF EXEMPLARY EMBODIMENTS

As used herein, the singular form of "a", "an", and "the" include plural references unless the context clearly dictates otherwise. As used herein, the statement that two or more parts or components are "coupled" shall mean that the parts are joined or operate together either directly or indirectly, i.e., through one or more intermediate parts or components, so long as a link occurs. As used herein, "directly coupled" means that two elements are directly in contact with each other. As used herein, "fixedly coupled" or "fixed" means that two components are coupled so as to move as one while maintaining a constant orientation relative to each other.

As used herein, the word "unitary" means a component is created as a single piece or unit. That is, a component that includes pieces that are created separately and then coupled together as a unit is not a "unitary" component or body. As employed herein, the statement that two or more parts or components "engage" one another shall mean that the parts exert a force against one another either directly or through one or more intermediate parts or components. As employed herein, the term "number" shall mean one or an integer greater than one (i.e., a plurality).

Directional phrases used herein, such as, for example and without limitation, top, bottom, left, right, upper, lower, front, back, and derivatives thereof, relate to the orientation of the elements shown in the drawings and are not limiting upon the claims unless expressly recited therein.

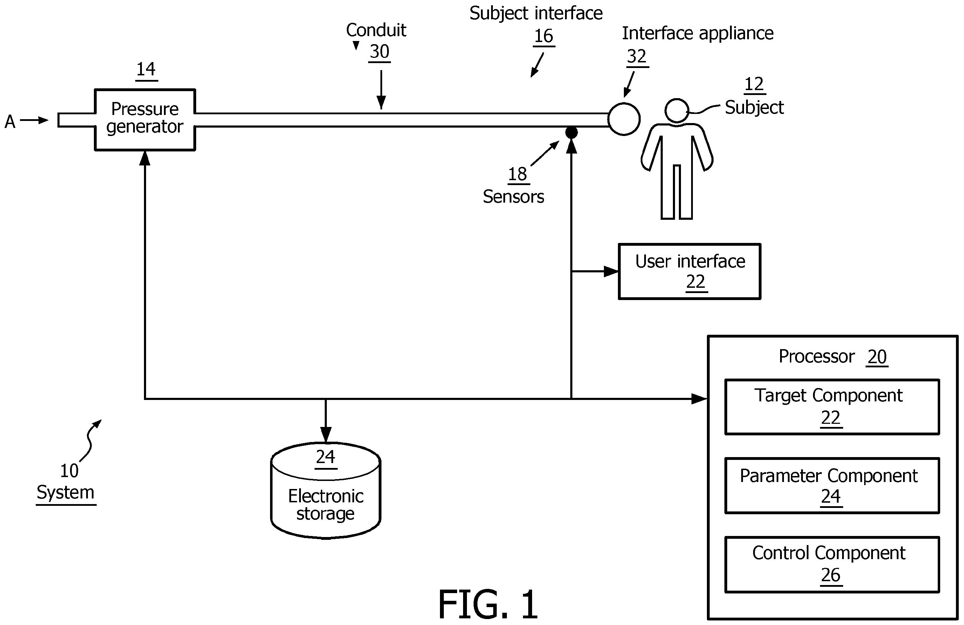

FIG. 1 schematically illustrates an exemplary embodiment of a system 10 configured to insufflate and exsufflate (hereafter "inexsufflate") a subject 12. System 10 provides an effective way to ensure that a desired lung volume is recruited when using mechanical in-exsufflation therapy. System 10 includes a volume operation mode wherein a clinician selects a desired target respiratory volume to achieve the therapy objective. The volume operation mode of system 10 eliminates the uncertainty of estimating a pressure and time parameter for the desired lung recruitment using mechanical in-exsufflation. In some embodiments, system 10 comprises one or more of a pressure generator 14, a subject interface 16, one or more sensors 18, a processor 20, a user interface 22, electronic storage 24, and/or other components.

Pressure generator 14 is configured to provide a pressurized flow of breathable gas for delivery to the airway of subject 12 (inflow to subject 12) and/or to draw gas from the airway (outflow from subject 12) of subject 12 (e.g., to inexsufflate). Pressure generator 14 may be configured such that one or more gas parameters of the pressurized flow of breathable gas are controlled in accordance with an in-exsufflation therapy regime to inexsufflate subject 12. The one or more gas parameters may include, for example, one or more of volume, pressure, flow rate, time, humidity, velocity, acceleration, and/or other parameters. In some embodiments, pressure generator 14 is a device dedicated to mechanical in-exsufflation. In some embodiments, pressure generator 14 is a ventilator and/or positive airway pressure device configured to provide therapy other than and/or in addition to in-exsufflation.

Pressure generator 14 receives a flow of gas from a gas source, such as the ambient atmosphere, as indicated by arrow A and elevates the pressure of that gas for delivery to the airway of a patient. Pressure generator 14 is any device, such as, for example, a pump, blower, piston, or bellows, that is capable of elevating the pressure of the received gas for delivery to a patient. The present disclosure also contemplates that gas other than ambient atmospheric air may be introduced into system 10 for delivery to the patient. In such embodiments, a pressurized canister or tank of gas containing air, oxygen, and/or another gas may supply the intake of pressure generator 14.

Pressure generator 14 may comprise one or more valves for controlling the pressure and/or flow direction of gas in pressure generator 14, a manifold defining the gas flow path in pressure generator 14, and/or other components. The present disclosure also contemplates controlling the operating speed of the blower, for example, either alone or in combination with such valves and/or the manifold, to control the pressure/flow of gas provided to and/or drawn from the patient.

By way of a non-limiting example, pressure generator 14 may be configured to adjust the parameters of the pressurized flow of breathable gas in accordance with an in-exsufflation therapy regime. In one embodiment, the therapy regime may dictate that the pressurized flow of breathable gas is delivered to the airway of subject 12 at a first pressure and time corresponding to the target volume during insufflation. The first volume is sufficiently high enough that the lung volume of subject 12 is at least partially recruited during insufflation.

After insufflation, pressure generator 14 may reduce the pressure of the pressurized flow of breathable gas with sufficient abruptness that expiratory flow through the airway of subject 12 is sufficient to remove mucus and/or other debris from the airway and/or lungs of subject 12. The pressure may be reduced from the first pressure level to a second pressure level that is substantially lower than the first pressure level. The second pressure level may, for example, be a negative pressure, below atmospheric pressure. After expiration is complete, pressure generator 14 may return the pressure of the pressurized flow of breathable gas to the first pressure level to facilitate another inspiration in preparation for another in-exsufflation. After a series of in-exsufflations, in-exsufflation may be ceased.

Subject interface 16 is configured to deliver the pressurized flow of breathable gas to the airway of subject 12. As such, subject interface 16 comprises conduit 30, interface appliance 32, and/or other components. Conduit 30 is configured to convey the pressurized flow of gas to interface appliance 32. Conduit 30 may be a flexible length of hose, or other conduit, that places interface appliance 32 in fluid communication with pressure generator 14. Interface appliance 32 is configured to deliver the flow of gas to the airway of subject 12. In some embodiments, interface appliance 32 is configured to be removably coupled with conduit 30 and/or other conduits and/or interface appliances being used to deliver respiratory therapy to subject 12. In some embodiments, interface appliance 32 is non-invasive. As such, interface appliance 32 non-invasively engages subject 12. Non-invasive engagement comprises removably engaging an area (or areas) surrounding one or more external orifices of the airway of subject 12 (e.g., nostrils and/or mouth) to communicate gas between the airway of subject 12 and interface appliance 32. Some examples of non-invasive interface appliance 32 may comprise, for example, a nasal cannula, a nasal mask, a nasal/oral mask, a full face mask, a total face mask, or other interface appliances that communicate a flow of gas with an airway of a subject. In some embodiments, interface appliance 32 is invasive. Some examples of invasive interface appliances that may comprise interface appliance 32 are endotracheal tubes, tracheostomy tubes, and or other devices. The present disclosure is not limited to these examples, and contemplates delivery of the flow of gas to the subject using any interface appliance.

Although subject interface 16 is illustrated in FIG. 1 as a single-limbed interface for the delivery of the flow of gas to the airway of the subject, this is not intended to be limiting. The scope of this disclosure comprises double-limbed circuits having a first limb configured to both provide the flow of gas to the airway of the subject, and a second limb configured to selectively exhaust gas (e.g., to exhaust exhaled gases).

Sensors 18 are configured to generate output signals conveying information related to one or more parameters of the pressurized flow of breathable gas. The parameters may include parameters related to gas within subject interface 16 and/or other components of system 10, parameters related to the respiration of subject 12, parameters related to the in-exsufflation therapy regime, and/or other parameters. For example, the one or more parameters may include one or more of a flow rate, a volume, a pressure, a composition (e.g., concentration(s) of one or more constituents), a temperature, a humidity, an acceleration, a velocity, and/or other parameters. In some embodiments, sensors 18 include a volume sensor, a flow rate sensor, a pressure sensor and/or other sensors.

Sensors 18 may comprise one or more sensors that measure such parameters directly (e.g., through fluid communication with the flow of gas in subject interface 16). Sensors 18 may comprise one or more sensors that generate output signals related to one or more parameters of the flow of gas indirectly. For example, one or more of sensors 18 may generate an output based on an operating parameter of pressure generator 14 (e.g., a valve driver or motor current, voltage, rotational velocity, and/or other operating parameters), and/or other information. Although sensors 18 are illustrated at a single location within (or in communication with) conduit 30 between interface appliance 32 and pressure generator 14, this is not intended to be limiting. Sensors 18 may include sensors disposed in a plurality of locations, such as for example, within pressure generator 14, within (or in communication with) interface appliance 32, and/or other locations.

Processor 20 is configured to provide information processing capabilities in system 10. As such, processor 20 may comprise one or more of a digital processor, an analog processor, a digital circuit designed to process information, an analog circuit designed to process information, a state machine, and/or other mechanisms for electronically processing information. Although processor 20 is shown in FIG. 1 as a single entity, this is for illustrative purposes only. In some implementations, processor 20 may comprise a plurality of processing units. These processing units may be physically located within the same device (e.g., pressure generator 14), or processor 20 may represent processing functionality of a plurality of devices operating in coordination.

As shown in FIG. 1, processor 20 is operatively connected to one or more sensors 18 and pressure generator 14. Processor 20 is configured by computer readable instructions and cooperable with the sensors 18 and pressure generator 14. Processor 20 is configured to execute one or more computer program components. The one or more computer program components may comprise one or more of a target component 22, a parameter component 24, a control component 26, and/or other components. Processor 20 may be configured to execute components 22, 24, and/or 26 by software; hardware; firmware; some combination of software, hardware, and/or firmware; and/or other mechanisms for configuring processing capabilities on processor 20.

It should be appreciated that although components 22, 24, and/or 26 are illustrated in FIG. 1 as being co-located within a single processing unit, in implementations in which processor 20 comprises multiple processing units, one or more of components 22, 24, and/or 26 may be located remotely from the other components. The description of the functionality provided by the different components 22, 24, and/or 26 described below is for illustrative purposes, and is not intended to be limiting, as any of components 22, 24, and/or 26 may provide more or less functionality than is described. For example, one or more of components 22, 24, and/or 26 may be eliminated, and some or all of its functionality may be provided by other components 22, 24, and/or 26. As another example, processor 20 may be configured to execute one or more additional components that may perform some or all of the functionality attributed below to one of components 22, 24, and/or 26.

Target component 22 is configured to obtain a target respiratory volume for subject 12. The target respiratory volume for subject 12 may comprise a target respiratory volume for an insufflation phase of in-exsufflation therapy. In some embodiments, the target respiratory volume includes one or more of, a target inspiratory reserve volume, a target inspiratory capacity, a target forced vital capacity, a target upper inflection point, a target expiratory reserve volume, a target total lung volume, and/or other target respiratory volumes. The target respiratory volume may be obtained from a user, one or more processors 20, electronic storage 24, and/or other sources. The user may include subject 12, a clinician, nurse, caregiver, and/or other users. The target respiratory volume may be based on and/or determined using one or more of biological information, a previously performed lung volume test and/or spirometry data, other information for achieving a specific therapy objective, and/or other relevant information. In some embodiments, target component 22 may be configured to obtain a target forced vital capacity based on the age, height, mass, sex, and/or ethnicity of subject 12. In some embodiments, target component 22 may be configured to obtain a target upper inflection point based on the forced vital capacity of subject 12.

Parameter component 24 is configured to determine one or more parameters within system 10. The one or more parameters within system 10 may comprise gas parameters related to the pressurized flow of breathable gas, breathing parameters of subject 12, parameters related to the in-exsufflation therapy, and/or other parameters. Parameter component 24 is configured to determine the one or more parameters based on the output signals of sensors 18, and/or other information. The information determined by parameter component 24 may be used by control component 26 for controlling pressure generator 14, used by other components of processor 20, stored in electronic storage 24, displayed by user interface 22, and/or used for other purposes.

The one or more gas parameters determined by parameter component 24 may include one or more of a volume, a pressure, a flow rate, a time, a humidity, a velocity, an acceleration, and/or other gas parameters of the pressurized flow of breathable gas determined based on output signals from one or more sensors 18. In some embodiments, the one or more gas parameters determined by parameter component 24 may be related to the target respiratory volume (e.g., obtained by target component 22), and may include and/or correspond to insufflation inflow of the pressurized flow of breathable gas and include one or more of an insufflation volume, insufflation pressure, insufflation flow rate, insufflation time, insufflation humidity, insufflation velocity, insufflation acceleration, and/or other gas parameters.

In some embodiments, parameter component 24 may be configured to determine one or more breathing parameters that correspond to various actual compartmentalized lung volumes. The compartmentalized lung volumes may include for example a inspiratory capacity, a forced vital capacity, a upper inflection point, a expiratory reserve volume, a total lung volume, and/or other compartmentalized lung volumes of subject 12. Parameter component 24 is configured to determine one or more breathing parameters that correspond to one or more compartmentalized lung volumes based on the output signals of sensors 18 (e.g., direct measurement of the volume), other determined parameters such as the pressure of the pressurized flow of breathable gas, the time of the pressurized flow of breathable gas, the flow rate of the pressurized flow of breathable gas during in-exsufflation, and/or other information. The one or more breathing parameters determined by parameter component 24 based on the output signals during the first respiratory cycle and/or any subsequent respiratory cycles may include one or more of inspiratory reserve volume, a inspiratory capacity, a forced vital capacity, a upper inflection point, a expiratory reserve volume, a total lung volume, and/or other breathing parameters of subject 12 determined during a respiratory cycle of in-exsufflation.

In some embodiments, parameter component 24 is configured to determine one or more breathing parameters of the subject based on the output signals during an insufflation, pause, and/or exsufflation portions of the one or more respiratory cycles of the in-exsufflation therapy. For example, the inspiratory reserve volume and/or inspiratory capacity of subject 12 may be determined by parameter component 24 during the insufflation of a respiratory cycle of the in-exsufflation therapy. The expiratory reserve volume of subject 12 may be determined during the exsufflation of a respiratory cycle of the in-exsufflation therapy. A forced vital capacity, an upper inflection point, a total lung volume, and/or other breathing parameters of subject 12 may be determined during any combination of one or more of the insufflation, exsufflation, and/or pause of a respiratory cycle of the in-exsufflation therapy. In some implementations, the forced vital capacity of subject 12 is determined both during the insufflation and pause of the first respiratory cycle and during the exsufflation of the first respiratory cycle.

Control component 26 is configured to deliver in-exsufflation therapy. Control component 26 is configured to deliver in-exsufflation therapy by controlling pressure generator 14 to deliver the pressurized flow of breathable gas according to an in-exsufflation therapy regime. Control component 26 is configured to control pressure generator 14 based on the output signals from sensors 18, gas and/or breathing parameters determined by parameter component 24, information entered and/or selected by a user via user interface 22 (e.g., the target respiratory volume), and/or other information. In some embodiments, the in-exsufflation therapy regime specifies an insufflation volume, a maximum pressure, and/or other parameters of the pressurized flow of breathable gas.

In some embodiments, control component 26 is configured to control pressure generator 14 to reduce the pressure of the pressurized flow of breathable gas for exsufflation compared to the pressure during insufflation with sufficient abruptness that expiratory flow through the airway of subject 12 is sufficient to remove mucus and/or other debris from the airway and/or lungs of subject 12.

During a first respiratory cycle, control component 26 is configured to deliver in-exsufflation therapy according to an in-exsufflation therapy regime based on the gas parameters determined by parameter component 24. The first respiratory cycle includes a first insufflation and a first exsufflation. During a second respiratory cycle, control component 26 is configured to deliver in-exsufflation therapy according to an in-exsufflation therapy regime based on the breathing parameters of subject 12 determined by parameter component 24 and the target respiratory volume obtained by target component 22. The in-exsufflation therapy regime corresponding to the second respiratory cycle, based on the breathing parameters and the target respiratory volume, may be different than the in-exsufflation therapy regime corresponding to the first respiratory cycle based on the gas parameters.

In some embodiments, control component 26 is further configured to compare one or more of the breathing parameters of subject 12 to the target respiratory volume. Control component 26 may be configured to compare breathing parameters that correspond to the target respiratory volume. For example, control component 26 may be configured to compare a forced vital capacity of subject 12 determined during the first respiratory cycle, to a target forced vital capacity obtained by target component 22. For example, parameter component 24 may be configured to determine an upper inflection point using the breathing parameters determined during the first respiratory cycle and control component 26 may be configured to compare the determined upper inflection point to a target upper inflection point obtained by target component 22.

In some embodiments, control component 26 is configured to adjust an insufflation pressure and an insufflation time of the pressurized flow of breathable gas based on the comparison between one or more of the determined breathing parameters and the target respiratory volume. For example, if the forced vital capacity (e.g., a determined breathing parameter) is lower than the target forced vital capacity, control component 26 may increase the insufflation pressure and/or insufflation time of the pressurize flow of breathable gas. In some embodiments, the insufflation pressure and/or insufflation time are increased according to a predetermined pressure step profile. Control component 26 is configured to cease increasing the insufflation pressure and/or insufflation time and maintain the insufflation pressure and/or insufflation time for subsequent respiratory cycles responsive to the comparison by control component 26 indicating that an individual one of one or more breathing parameters reached the target respiratory volume.

In some embodiments, parameter component 24 and control component 26 are configured to repeat the determination of one or more breathing parameters of subject 12, the comparison of one or more of the breathing parameters to a target respiratory volume, and the adjustment of the in-exsufflation therapy based on the comparison for multiple respiratory cycles. For example, continuing with the example above, the forced vital capacity is determined by parameter component 24 during each of the multiple respiratory cycles of in-exsufflation therapy and the insufflation pressure and/or insufflation time for the subsequent breathing cycles is increased or decreased based on the determined forced vital capacity of the previous cycle and the target forced vital capacity.

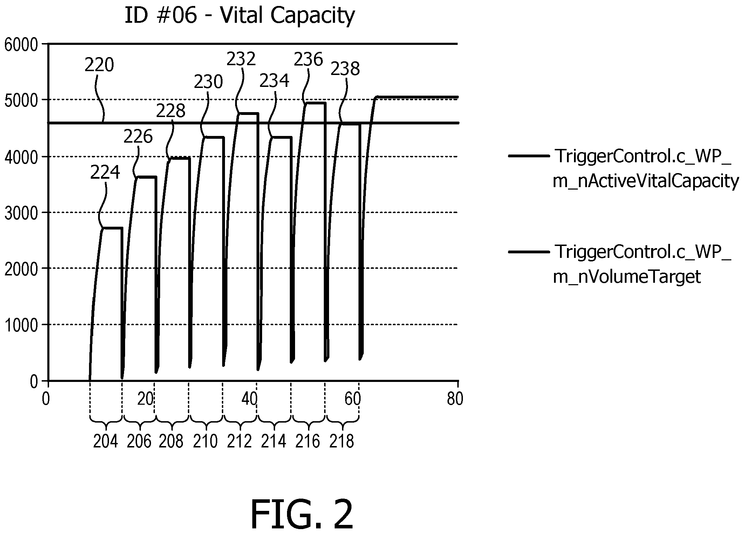

By way of further example FIG. 2 illustrates multiple respiratory cycles of in-exsufflation therapy delivered based on the determined forced vital capacity (e.g., determined breathing parameters) of each previous respiratory cycle and the target forced vital capacity (e.g., the target respiratory volume). In FIG. 2, control component 26 (FIG. 1) is configured to deliver in-exsufflation therapy for multiple respiratory cycles 204, 206, 208, 210, 212, 214, 216, and 218 and parameter component 24 is configured to determine breathing parameters, 224, 226, 228, 230, 232, 234, 236, and 238. Based on determined breathing parameters 224, 226, 228, 230, 232, 234, 236, and 238, control component 26 is configured to adjust the insufflation pressure and insufflation time in order to reach and/or maintain the target respiratory volume 220. Parameter component 24 determines one or more individual ones of breathing parameters, 224, 226, 228, 230, 232, 234, 236, and/or 238 based on information determined during the individual ones of respiratory cycles, 204, 206, 208, 210, 212, 214, 216, or 218 immediately previous, and adjusts the insufflation pressure and insufflation time for the subsequent respiratory cycles, such that one or more individual ones of breathing parameters, 224, 226, 228, 230, 232, 234, 236, and/or 238 satisfies the target respiratory volume. In some embodiments, satisfying the target respiratory volume means that the individual breathing parameter is equal and/or close to the target volume such that the therapy objective is achieved. In some embodiments, the target volume may not be satisfied if a preset maximum pressure for the pressurized flow of breathable gas is reached prior to the target respiratory volume. For example, if a user sets a maximum pressure via user interface 22, control component 26 will not increase the insufflation pressure beyond the maximum pressure even if one or more of the determined breathing parameters has not reached the target respiratory volume.

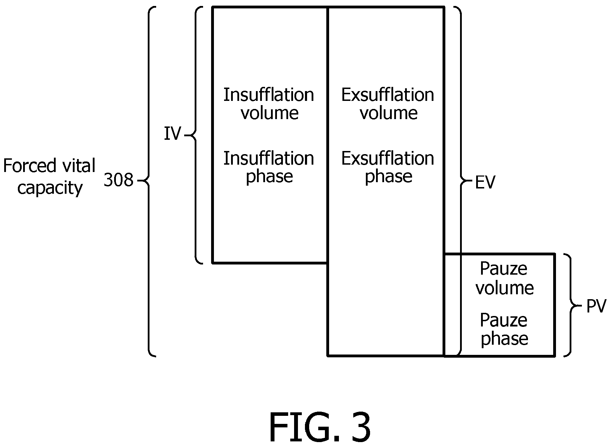

As an example of the determined breathing parameters, FIG. 3 illustrates the determination of a forced vital capacity 308 during a respiratory cycle of in-exsufflation therapy. In some embodiments, parameter component 24 is configured to determine the forced vital capacity 308 of subject 12 based on the output signals during the first insufflation and a first pause and/or based on the output signals during the first exsufflation. The forced vital capacity may be determined based on a insufflation volume 302 plus a pause volume 306 (e.g., FVC= IV+PV) and/or based on a exsufflation volume 304 (e.g., FVC=EV). During an ideal in-exsufflation therapy regime, insufflation volume 302 plus pause volume 306 should equal exsufflation volume 304. In some embodiments, a large difference between these two measurements is a good indication of a potential problem and can be used to improve the efficacy of the in-exsufflation therapy.

For example, a significantly large forced vital capacity measured based on an insufflation volume plus a pause volume compared to a target forced vital capacity and/or a forced vital capacity measured based on the exsufflation volume suggests that there is a large user interface leak during the insufflation. This information can be used to readjust the user's interface to minimize the insufflation leak and improve the efficacy of the in-exsufflation therapy. By way of further example, a significantly reduced forced vital capacity measured based on the exsufflation volume compared to the target forced vital capacity and/or forced vital capacity measured based on the insufflation volume plus pause volume suggests that there is an obstruction during the exsufflation cycle due to the upper airway collapse. This information may assist clinicians and/or other users to adjust the exsufflation pressure and/or time to resolve the obstruction.

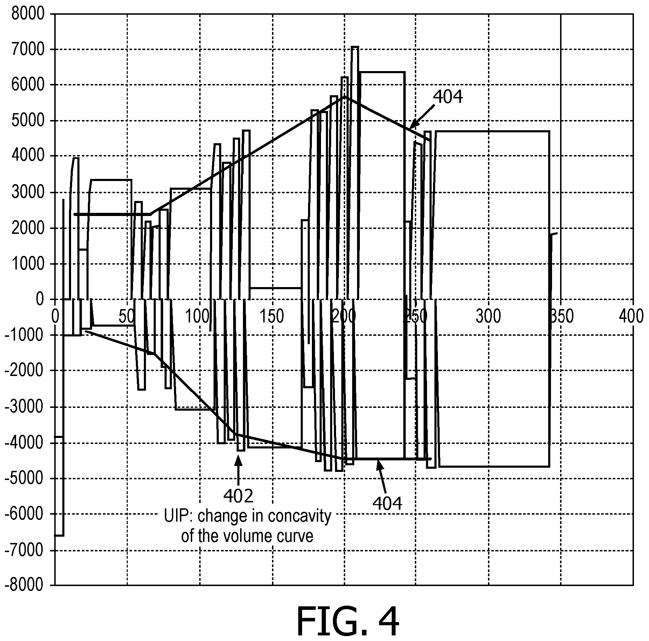

FIG 4 illustrates the estimation of an upper inflection point (e.g., one of the determined breathing parameters) on a pressure volume curve. In some embodiments, parameter component 24 (FIG. 1) is configured to determine a breathing parameter using other breathing parameters determined during the first respiratory cycle by parameter component 24. Control component 26 is configured to compare the determined breathing parameter to the target respiratory volume. For example, upper inflection point 402 is determined from the breathing parameters determined during the in-exsufflation therapy and displayed in FIG. 4 as a pressure volume curve 404. Upper inflection point 402 is determined by determining where the pressure volume curve changes concavity. A change in concavity, for example, indicates a clinical regional lung over distention. In some embodiments, the pressure of the pressurized flow of breathable gas increases linearly. At upper inflection point 402, the increase in volume, as shown in pressure volume curve 404, no longer corresponds to the increase in pressure and the concavity of the pressure volume curve changes. Upper inflection point 402 is theoretically found when the second derivative of pressure volume curve 404 becomes zero. In some embodiments, upper inflection point 402 is found when the second derivative of the pressure volume curve becomes an arbitrary value used to define a pseudo upper inflection point based on the clinical needs of the subject 12.

For example, FIG. 4 shows multiple cycles of volume measurement at a stepwise insufflation pressure increase. An average of 3 and/or 4 volume measurement samples at the specific insufflation pressure is used to estimate an upper inflection point (i.e., UIP). An UIP and/or pseudo UIP may be estimated with a single in-exsufflation cycle and/or multiple in-exsufflation cycles. An UIP and/or pseudo UIP can also be estimated from either the insufflation cycle and/or the exsufflation cycle based on its respective measured pressure and volume curves. A generic UIP calculation can be described as follows; Volume_Lung (p) = Pressure_applied. The measured volume and pressure can be curved fitted using typical regression models. For example, logarithmatic, exponential, polynomial, and/or other regression models. Calculating a UIP can be described as; Volume_Lung (p)"= 0 (e.g., setting the second derivative equal to zero or an arbitrary constant). In some implementations, the clinician and/or other users can use this UIP producing pressure and volume to set either the maximum peak insufflation pressure and/or target respiratory volume to minimize the risk of the lung regional over distention.

Returning to FIG. 1, user interface 22 is configured to provide an interface between system 10 and subject 12 and/or other users through which subject 12 and/or other users may provide information to and receive information from system 10. Other users may comprise a caregiver, a doctor, a decision maker, and/or other users. This enables data, cues, results, and/or instructions and any other communicable items, collectively referred to as "information," to be communicated between a user (e.g., subject 12) and one or more of pressure generator 14, processor 20, and/or other components of system 10. In some embodiments, a target respiratory volume for system 10 is provided by a user through user interface 22. Examples of interface devices suitable for inclusion in user interface 22 comprise a keypad, buttons, switches, a keyboard, knobs, levers, a display screen, a touch screen, speakers, a microphone, an indicator light, an audible alarm, a printer, a tactile feedback device, and/or other interface devices. In one embodiment, user interface 22 comprises a plurality of separate interfaces. In one embodiment, user interface 22 comprises at least one interface that is provided integrally with pressure generator 14.

It is to be understood that other communication techniques, either hardwired or wireless, are also contemplated by the present disclosure as user interface 22. For example, the present disclosure contemplates that user interface 22 may be integrated with a removable storage interface provided by electronic storage 24. In this example, information may be loaded into system 10 from removable storage (e.g., a smart card, a flash drive, a removable disk, etc.) that enables the user(s) to customize the implementation of system 10. Other exemplary input devices and techniques adapted for use with system 10 as user interface 22 comprise, but are not limited to, an RS-232 port, RF link, an IR link, modem (telephone, cable, or other). In short, any technique for communicating information with system 10 is contemplated by the present disclosure as user interface 22.

In some embodiments, electronic storage 24 comprises electronic storage media that electronically stores information. The electronic storage media of electronic storage 24 may comprise one or both of system storage that is provided integrally (i.e., substantially non-removable) with system 10 and/or removable storage that is removably connectable to system 10 via, for example, a port (e.g., a USB port, a firewire port, etc.) or a drive (e.g., a disk drive, etc.). Electronic storage 24 may comprise one or more of optically readable storage media (e.g., optical disks, etc.), magnetically readable storage media (e.g., magnetic tape, magnetic hard drive, floppy drive, etc.), electrical charge-based storage media (e.g., EPROM, RAM, etc.), solid-state storage media (e.g., flash drive, etc.), and/or other electronically readable storage media. Electronic storage 24 may store software algorithms, information determined by processor 20, information received via user interface 22, and/or other information that enables system 10 to function properly. Electronic storage 24 may be (in whole or in part) a separate component within system 10, or electronic storage 24 may be provided (in whole or in part) integrally with one or more other components of system 10 (e.g., user interface 22, processor 20, etc.).

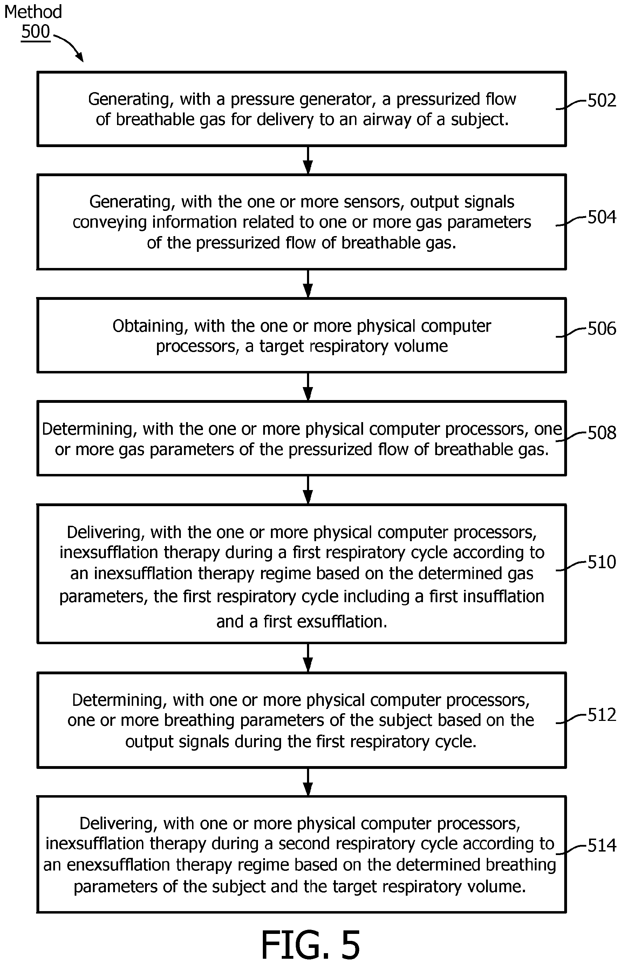

FIG. 5 illustrates a method 500 for inexsufflating a subject with an in-exsufflation system. The in-exsufflation system comprises a pressure generator, one or more sensors, one or more physical computer processors, and/or other components. The operations of method 500 presented below are intended to be illustrative. In some embodiments, method 500 may be accomplished with one or more additional operations not described, and/or without one or more of the operations discussed. Additionally, the order in which the operations of method 500 are illustrated in FIG. 5 and described below is not intended to be limiting.

In some embodiments, method 500 may be implemented in one or more processing devices (e.g., a digital processor, an analog processor, a digital circuit designed to process information, an analog circuit designed to process information, a state machine, and/or other mechanisms for electronically processing information). The one or more processing devices may include one or more devices executing some or all of the operations of method 500 in response to instructions stored electronically on an electronic storage medium. The one or more processing devices may include one or more devices configured through hardware, firmware, and/or software to be specifically designed for execution of one or more of the operations of method 500.

At an operation 502, a pressurized flow of breathable gas for delivery to the airway of a subject is generated. In some embodiments, operation 502 is performed by a pressure generator the same as or similar to pressure generator 14 (shown in FIG. 1 and described herein).

At an operation 504, output signals are generated conveying information related to one or more gas parameters of the pressurized flow of breathable gas. In some embodiments, operation 504 is performed by sensors the same as or similar to sensors 18 (shown in FIG. 1 and described herein).

At operation 506, a target respiratory volume is obtained. In some embodiments, operation 506 is performed by a physical computer processor the same as or similar to processor 20 (shown in FIG. 1 and described herein).

At operation 508, one or more gas parameters of the pressurized flow of breathable gas are determined. In some embodiments, operation 508 is performed by a physical computer processor the same as or similar to processor 20 (shown in FIG. 1 and described herein).

At operation 510, in-exsufflation therapy during a first respiratory cycle according to an in-exsufflation therapy regime based on the determined gas parameters is delivered. The first respiratory cycle included a first insufflation and a first exsufflation. In some embodiments, operation 510 is performed by a physical computer processor the same as or similar to processor 20 (shown in FIG. 1 and described herein).

At operation 512, one or more breathing parameters of the subject are determined based on the output signals during the first respiratory cycle. In some embodiments, the one or more breathing parameters of the subject further include one or more of a forced vital capacity, an inspiratory reserve volume, an inspiratory capacity, an expiratory reserve volume, or an upper inflection point. In some embodiments, operation 512 is performed by a physical computer processor the same as or similar to processor 20 (shown in FIG. 1 and described herein).

At operation 514, in-exsufflation therapy during a second respiratory cycle according to an in-exsufflation therapy regime based on the determined breathing parameters of the subject and the target respiratory volume is delivered. In some embodiments, operation 514 is performed by a physical computer processor the same as or similar to processor 20 (shown in FIG. 1 and described herein).

In some embodiments, in addition to and/or as part of operation 514, one or more of the determined breathing parameters are compared to the target respiratory volume; and an insufflation pressure and/or an insufflation time of the pressurized flow of breathable gas are adjusted based on the comparison between one of more of the determined breathing parameters and the target respiratory volume. In some embodiments, this portion of operation 514 is performed by a physical computer processor the same as or similar to processor 20 (shown in FIG. 1 and described herein).

In some embodiments, in addition to and/or as part of operation 514, the determination of one or more breathing parameters of the subject based on the output signals from a previous respiratory cycle may be repeated; and the delivery of in-exsufflation therapy for one or more respiratory cycles according to the in-exsufflation therapy regime based on one or more of the determined breathing parameters of the subject and the target respiratory volume may be repeated. The determination and delivery are repeated such that and/or until one or more of the determined breathing parameters of the subject satisfies the target respiratory volume. In some embodiments, this portion of operation 514 is performed by a physical computer processor the same as or similar to processor 20 (shown in FIG. 1 and described herein).

In some embodiments, in addition to and/or as part of operation 514, a forced vital capacity of the subject based on the output signals during the first insufflation and a first pause of the first respiratory cycle and/or based on the output signals during the first exsufflation of the first respiratory cycle, is determined. In some embodiments, this portion of operation 514 is performed by a physical computer processor the same as or similar to processor 20 (shown in FIG. 1 and described herein).

In the claims, any reference signs placed between parentheses shall not be construed as limiting the claim. The word "comprising" or "including" does not exclude the presence of elements or steps other than those listed in a claim. In a device claim enumerating several means, several of these means may be embodied by one and the same item of hardware. The word "a" or "an" preceding an element does not exclude the presence of a plurality of such elements. In any device claim enumerating several means, several of these means may be embodied by one and the same item of hardware. The mere fact that certain elements are recited in mutually different dependent claims does not indicate that these elements cannot be used in combination.

Although the description provided above provides detail for the purpose of illustration based on what is currently considered to be the most practical and preferred embodiments, it is to be understood that such detail is solely for that purpose and that the disclosure is not limited to the expressly disclosed embodiments, but, on the contrary, is intended to cover modifications and equivalent arrangements that are within the scope of the appended claims. For example, it is to be understood that the present disclosure contemplates that, to the extent possible, one or more features of any embodiment can be combined with one or more features of any other embodiment.

## Claims

1. A system (10) configured to in-exsufflate a subject, the system comprising:
a pressure generator (14) for generating a pressurized flow of breathable gas for delivery to an airway of the subject;
one or more sensors (18) for generating output signals conveying information related to one or more gas parameters of the pressurized flow of breathable gas;
one or more physical computer processors (20) operatively connected to the one or more sensors and the pressure generator, configured to
obtain a target respiratory volume;
determine one or more gas parameters of the pressurized flow of breathable gas;
deliver in-exsufflation therapy during a first respiratory cycle according to an in-exsufflation therapy regime based on the determined gas parameters, the first respiratory cycle including a first insufflation and a first exsufflation;
determine one or more breathing parameters of the subject based on the output signals during the first respiratory cycle; and
deliver in-exsufflation therapy during a second respiratory cycle according to an in-exsufflation therapy regime based on the determined breathing parameters of the subject and the target respiratory volume,
wherein the one or more physical computer processors are further configured such that the determined one or more breathing parameters of the subject include one or more of a forced vital capacity, an inspiratory reserve volume, an inspiratory capacity, an expiratory reserve volume, or an upper inflection point.

2. The system of claim 1, wherein the one or more physical computer processors are further configured to:
compare one or more of the determined breathing parameters to the target respiratory volume; and
adjust an inhalation pressure and an inhalation time of the pressurized flow of breathable gas based on the comparison between one or more of the determined breathing parameters and the target respiratory volume.

3. The system of claim 1, wherein the one or more physical computer processors are further configured to:
repeat the determination of one or more breathing parameters of the subject based on the output signals from a previous respiratory cycle;
repeat the delivery of in-exsufflation therapy for one or more respiratory cycles according to the in-exsufflation therapy regime, based on one or more of the determined breathing parameters of the subject and the target respiratory volume, such that one or more of the determined breathing parameters of the subject satisfies the target respiratory volume.

4. The system of claim 1, wherein the first respiratory cycle further includes a first pause and the means for determining one or more breathing parameters of the subject is further configured to determine the forced vital capacity of the subject based on the output signals during the first insufflation and first pause of the first respiratory cycle and/or based on the output signals during the first exsufflation of the first respiratory cycle.

## Patentansprüche

1. Ein System (10) für die In-Exsufflation eines Patienten, wobei das System Folgendes umfasst:
einen Druckgenerator (14), der den Atemwegen eines Patienten einen druckbeaufschlagten Atemluftstrom zuleitet;
mindestens einen Sensor (18), der Ausgangssignale erzeugt, die Informationen über den mindestens einen Luftparameter des druckbeaufschlagten Atemluftstroms übermitteln;
mindestens einen physischen Computerprozessor (20), der mit dem mindestens einen Sensor und dem Druckgenerator verbunden ist und ein Zielatemvolumen abruft; Ermitteln mindestens eines Luftparameters des druckbeaufschlagten Atemluftstroms;
Bereitstellen einer In-Exsufflationstherapie während des ersten Atemzyklus im Rahmen eines In-Exsufflationstherapie-Systems anhand der ermittelten Luftparameter,
wobei der erste Atmungszyklus eine erste Insufflation und eine erste Exsufflation umfasst;
Ermitteln mindestens eines Atmungsparameters des Patienten beruhend auf den Ausgangssignalen für den ersten Atmungszyklus; und
Bereitstellen einer In-Exsufflationstherapie während des zweiten Atemzyklus im Rahmen eines In-Exsufflationstherapie-Systems anhand der ermittelten Atmungsparameter des Patienten und des Zielatemvolumens ,
wobei der mindestens eine physische Computerprozessor zudem so konfiguriert ist, dass der mindestens eine ermittelte Atmungsparameter für den Patienten mindestens einen der folgenden Aspekte umfasst: forcierte Vitalkapazität, Inspirationsreservevolumen, Inspirationskapazität, exspiratorisches Reservevolumen oder oberer Wendepunkt.

2. Das System gemäß Anspruch 1, wobei der mindestens eine physische Computerprozessor zudem folgende Schritte durchführt:
Vergleichen mindestens eines der ermittelten Atmungsparameter mit dem Zielatemvolumen; und
Einstellen des Inhalationsdrucks und der Inhalationsdauer des druckbeaufschlagten Atemluftstroms beruhend auf dem Vergleich zwischen mindestens einem der ermittelten Atmungsparameter und dem Zielatemvolumen.

3. Das System gemäß Anspruch 1, wobei der mindestens eine physische Computerprozessor zudem folgende Schritte durchführt:
Wiederholen des Ermittelns mindestens eines Atmungsparameters des Patienten beruhend auf den Ausgangssignalen eines vorherigen Atmungszyklus;
Wiederholen des Bereitstellens der In-Exsufflationstherapie für mindestens einen Atemzyklus im Rahmen des In-Exsufflationstherapie-Systems beruhend auf mindestens einem der ermittelten Atmungsparameter für den Patienten und dem Zielatemvolumen, sodass mindestens einer der ermittelten Atmungsparameter für den Patienten dem Zielatemvolumen entsprechen.

4. Das System gemäß Anspruch 1, wobei der erste Atmungszyklus zudem eine erste Pause umfasst, und wobei die Vorrichtungen zum Ermitteln mindestens eines Atmungsparameters für den Patienten zudem die forcierte Vitalkapazität des Patienten beruhend auf den Ausgangssignalen während der ersten Insufflation und der ersten Pause des ersten Atmungszyklus und/oder beruhend auf den Ausgangssignalen während der ersten Exsufflation des ersten Atmungszyklus ermittelt.

## Revendications

1. Système (10) conçu pour insuffler-exsuffler un sujet, ledit système comprenant :
un générateur de pression (14) destiné à la génération d'un écoulement pressurisé de gaz respirable destiné à être délivré à une voie aérienne du sujet ;
au moins un capteur (18) destiné à la génération des signaux de sortie véhiculant des informations relatives à au moins un paramètre de gaz de l'écoulement pressurisé de gaz respirable ;
au moins un processeur informatique physique (20) connecté de manière fonctionnelle à l'au moins un capteur et au générateur de pression,
conçu pour obtenir un volume respiratoire cible ;
la détermination d'au moins un paramètre de gaz de l'écoulement pressurisé de gaz respirable ;
l'administration d'une thérapie par exsufflation lors d'un premier cycle respiratoire selon un régime de thérapie par insufflation en fonction des paramètres de gaz déterminés, le premier cycle respiratoire comprenant une première insufflation et une première exsufflation ;
la détermination d'au moins un paramètre respiratoire du sujet en fonction des signaux de sortie lors du premier cycle respiratoire ; et
l'administration d'une thérapie par insufflation-exsufflation lors d'un second cycle respiratoire selon un régime de thérapie par insufflation-exsufflation en fonction des paramètres respiratoires déterminés du sujet et du volume respiratoire cible, dans lequel l'au moins un processeur informatique physique est en outre configuré de telle sorte que l'au moins un paramètre respiratoire déterminé du sujet comprend une capacité vitale forcée et/ou un volume de réserve inspiratoire et/ou une capacité inspiratoire et/ou un volume de réserve expiratoire et/ou un point d'inflexion supérieur.

2. Système selon la revendication 1, dans lequel l'au moins un processeur informatique physique est en outre configuré :
pour comparer l'au moins un des paramètres respiratoires déterminés au volume respiratoire cible ; et
pour ajuster une pression d'inhalation et un temps d'inhalation de l'écoulement pressurisé de gaz respirable en fonction de la comparaison entre l'au moins un des paramètres respiratoires déterminés et le volume respiratoire cible.

3. Système selon la revendication 1, dans lequel l'au moins un processeur informatique physique est en outre configuré :
pour répéter la détermination de l'au moins un paramètre respiratoire du sujet en fonction des signaux de sortie d'un cycle respiratoire précédent ;
pour répéter l'administration d'une thérapie par insufflation-exsufflation lors d'au moins un cycle respiratoire selon le régime de thérapie par insufflation-exsufflation en fonction de l'au moins un des paramètres respiratoires déterminés du sujet et du volume respiratoire cible de telle sorte que l'au moins un des paramètres respiratoires déterminés du sujet satisfait le volume respiratoire cible.

4. Système selon la revendication 1, dans lequel le premier cycle respiratoire comprend en outre une première pause et un moyen destiné à la détermination d'au moins un paramètre respiratoire du sujet est en outre configuré pour déterminer la capacité vitale forcée du sujet en fonction des signaux de sortie lors de la première insufflation et de la première pause du premier cycle respiratoire et/ou en fonction des signaux de sortie lors de la première exsufflation du premier cycle respiratoire.
